# EUROPEAN PATENT APPLICATION

(11) **EP 2 883 570 A1**
(43) Date of publication of application: **17.06.2015**
(21) Application number: 13197242.4
(22) Date of filing: 13.12.2013
(51) Int. Cl.: A61Q 5/08, A61K 8/43, A61Q 5/10

(54) **Powder hair lightening/bleaching composition**

(71) Applicant: Kao Corporation, Chuo-ku Tokyo 103-8210 (JP)
(72) Inventor: Feier-Iova, Ovidiu, 64283 Darmstadt (DE)
(74) Representative: Hoffmann Eitle

(57) **Abstract**

Present invention relates to a powder hair lightening/bleaching composition comprising two alkaline salts. Accordingly, the present invention is on a powder composition for lightening and/or bleaching hair comprising at least one alkalizing agent, at least one guanidine salt and at least one second alkaline salt selected from salts of carbonates, bicarbonates, phosphate and hydrogenphosphates with a cation selected from sodium, potassium or ammonium.

## Description

Present invention relates to a powder hair lightening/bleaching composition comprising two alkaline salts.

There are two main types of bleaching/lightening compositions which have been known for many years. The first is an anhydrous composition (usually powder) which comprises, as a rule, per salts and alkalizing agents and which is applied onto hair after mixing with an aqueous acidic composition comprising at least one oxidizing agent. Such lightening powders usually deliver satisfactory lightening but hair damage is very high and they are not compatible with standard hair dyestuffs. This has the disadvantage that two processes are needed to lighten and tone the hair. Additionally and importantly, they are not mild enough so that scalp/skin irritations are often observed so that cautions must be taken when applying directly onto skin in order to bleach hair at growth area.

The second type of lightener is usually an aqueous alkaline cream emulsion composition which is free of per salts and which is applied onto hair after mixing with an aqueous acidic composition comprising at least one oxidizing agent. Such lighteners are compatible with standard hair dyestuffs allowing simultaneous lightening and toning but do not deliver satisfactory lightening, especially on hair that has already been colored with oxidative dyes and therefore hair comprising parts colored previously with an oxidative dyeing composition and uncoloured parts cannot always be bleached homogeneously. In other words, such lightening technology only provides selective bleaching for natural and previously oxidatively coloured hair so that homogeneous colour appearance of such hair is not observed.

Therefore, aim of the present invention is to provide a hair lightening and/or bleaching composition which provides effective lightening of natural and previously coloured hair but causes less damage to the hair and milder to scalp than the presently available powder bleach compositions.

Present inventors have unexpectedly found out that a powder composition comprising two alkaline salts provide effective lightening with less damaging effect when used in combination with an alkalizing agent and after mixing with an aqueous acidic composition comprising at least one oxidizing agent.

Accordingly, the first object of the present invention is a powder composition for lightening and/or bleaching hair comprising at least one alkalizing agent, at least one guanidine salt and at least one second alkaline salt selected from salts of carbonates, bicarbonates, phosphate and hydrogenphosphates with a cation selected from sodium, potassium or ammonium.

The powder compositions preferably do not comprise the known per salts at a concentration of higher than 10% by weight and preferably 5% by weight and most preferably free of per salts, all values are calculated to the total of the powder composition.

Second object of the present invention is the use of the powder composition for lightening and/or bleaching human hair.

Third object of the present invention is a process for lightening and/or bleaching human hair wherein the powder composition is mixed with an aqueous composition which has an acidic pH and comprises at least one oxidizing agent, preferably hydrogen peroxide and is applied onto hair and after being left on the hair for 1 to 45 min is rinsed off from hair, and hair is optionally dried.

The fourth object of the present invention is kit for hair comprising two or more compositions wherein one of the compositions is the powder composition of the present invention and the other composition is an aqueous composition comprising at least one oxidizing agent having an acidic pH.

For example, EP 890 355 A1 discloses hair dyeing compositions comprising guanidium salt such as guanidium carbonate. The document does not disclose anything on combining the guanidium salt with another alkaline salt and/or with an alkalizing agent and it does not disclose any powder composition.

EP 642 783 discloses oxidative hair dyeing compositions comprising ammonium salts. Among the suitable salts ammonium carbonate and ammonium bicarbonate are mentioned. However, the document fails to disclose any combination with guanidium salts and does also not refer to any powder composition.

Furthermore, EP 560088 discloses powder bleaching composition comprising per salts and sodium carbonate. The document fails to disclose powder bleaching composition comprising two alkaline salts and does not disclose at all any guanidium salts.

The composition of the present invention comprises at least one guanidine salt. Suitable ones are guanidine carbonate, guanidine hydrochloride and guanidine phosphate. Particularly preferred is guanidine carbonate.

The concentration of at least one guanidium salt is in the range of 5 to 90%, preferably 10 to 75%, more preferably 15 to 60% and most preferably 15 to 50% by weight calculated to the total of the powder composition.

The composition of the present invention comprises at least one alkaline salt selected from salts of carbonate, bicarbonate, phosphates and hydrogen phosphates with a cation selected from sodium, potassium or ammonium. Suitable salts are sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate. Preferred are sodium bicarbonate, sodium carbonate, potassium bicarbonate, potassium carbonate, ammonium carbonate and ammonium bicarbonate. Particularly preferred are sodium bicarbonate, potassium bicarbonate and ammonium bicarbonate. Ammonium bicarbonate is the especially preferred alkaline salt.

The concentration of at least one alkaline salt is in the range of 5 to 90%, preferably 10 to 75%, more preferably 15 to 60% and most preferably 15 to 50% by weight calculated to the total of the powder composition.

It has also been found out that the weight ratio between the two salts may play a role in effective lightening of artificially colored hair. The weight ratio between guanidine salt and the second alkaline salt is in the range of 10:1 to 1:5, preferably 5:1 to 1:2, more preferably 3:1 to 1:2 and most preferably 2:1 to 1:1.

The powder compositions of the present invention comprise at least one alkalizing agent. Suitable and preferred alkalizing agent is sodium metasilicate and is comprised in the composition in the range of 1 to 20%, preferably 2 to 15% more preferably 2.5 to 12.5 and most preferably 5 to 10% by weight calculated to the total of the composition.

The powder composition of the present invention may comprise a powder, inert carrier material such as starch, diatomaceous earth, pyrogenic silicium dioxide.

Further, powder composition comprises thickening polymers, preferably selected from the group consisting of cellulose polymer compounds, alginate, polysaccarides and acrylic acid polymers, preferably methyl cellulose compounds, ethyl cellulose compounds, hydroxyethylcellulose compounds, methylhydroxyethylcellulose compounds, methylhydroxypropylcellulose compounds, carboxymethyl cellulose compounds, alginic acids, sodium alginates, ammonium alginates, calcium alginates, gum arabic, guar gum or xanthan gum, or acrylic acid polymers with molecular weights from about 1,250,000 to 4,000,000, alone or in combination with each other. The polymers are used in a total amount of 0.1 to 15 %, preferably from 0.2 to 10 %, and more preferably in an amount of from 0.5 to 7.5% by weight, calculated to total of the powder composition.

The powder composition can also comprise cationic polymers as conditioning and/or thickening agents. Preferred ones are those cationic cellulose type polymers know as Polymer JR type from Amerchol such as Polyquaternium 10 or cationic guar gum known with trade name Jaguar from Rhône-Poulenc and chemically for example Guar hydroxypropyl trimonium chloride. Furthermore, chitosan and chitin can also be included in the compositions as cationic natural polymers.

Furthermore, other cationic polymers known with their CTFA category name Polyquaternium are also suitable as conditioners and/or thickener. Typical examples of those Polyquaternium 1, Polyquaternium 2, Polyquaternium 4, Polyquaternium 5, Polyquaternium 6, Polyquaternium 7, Polyquaternium 8, Polyquaternium 9, Polyquaternium 11, Polyquaternium 12, Polyquaternium 13, Polyquaternium 14, Polyquaternium 15, Polyquaternium 16, Polyquaternium 17, Polyquaternium 18, Polyquaternium 19, Polyquaternium 20, Polyquaternium 22, Polyquaternium 24, Polyquaternium 27, Polyquaternium 28, Polyquaternium 29, Polyquaternium 30, Polyquaternium 31, Polyquaternium 32, Polyquaternium 33, Polyquaternium 34, Polyquaternium 35 and Polyquaternium 36, Polyquaternium-37, Polyquaternium 39, Polyquaternium 42, Polyquaternium 43, Polyquaternium 44, Polyquaternium 45, Polyquaternium 46, Polyquaternium 47, Polyquaternium 48, Polyquaternium-49, Polyquaternium 50, Polyquaternium 51, Polyquaternium 52, Polyquaternium 53, Polyquaternium 54, Polyquaternium 55, Polyquaternium 56, Polyquaternium 57, Polyquaternium 58, Polyquaternium 59, Polyquaternium 60, Polyquaternium 61, Polyquaternium 62, Polyquaternium 63, Polyquaternium 64, Polyquaternium 65, Polyquaternium 66, Polyquaternium 67, Polyquaternium 68, Polyquaternium 69, Polyquaternium-70, Polyquaternium 71, Polyquaternium 72, Polyquaternium 73, Polyquaternium 74, Polyquaternium 75, Polyquaternium 76, Polyquaternium 77, Polyquaternium 78, Polyquaternium-79, Polyquaternium 80, Polyquaternium 81, Polyquaternium 82, Polyquaternium 83, Polyquaternium 84, Polyquaternium 85, Polyquaternium 86 and Polyquaternium 87.

Typical concentration range for any of the cationic conditioners mentioned above can be 0.1 - 7.5% by weight, preferably 0.3 - 5% by weight and more preferably 0.5-2.5% by weight, calculated to total of the powder composition.

In a preferred embodiment of the present invention, the powder composition is in dust free powder form and comprises oily lipophilic ingredients such as vegetable oils, for example, jojoba oil or any other; petrolatum liquid paraffins, especially paraffinum perliquidum and parafiinum subliquidum; silicone oils such as dimethicones, dimethiconols and arylated silicones such as phenyl methicone, phenyl trimethicone, diphenyl dimethicone, diphenylsiloxy phenyl trimethicone, tetramethyl tetraphenyl trisiloxane, triphenyl trimethicone, tetramethly tetraphenyl trisiloxane and pentaphenyl trimethyl trisiloxane; hydropobic fatty acid esters such as octyl palmitate, isocetyl palmitate, isopropyl palmitate and octyl stearate, C₁₀- to C₃₆-fatty acid triglycerides, as well as their mixtures. In the case that the use is wished among those the most preferred ones are silicone oils, jojoba oil, fatty acid esters, paraffin oils, combinations of fatty acid esters and paraffin oils. Fatty acid esters and/or paraffin oils and/or silicone oils are particularly preferred. Concentration of these oily lipophilic compounds are used in a total amount of about 1 to 30% by weight, preferably 2.5 to 25%, more preferably 5 to 20% and most preferably 7.5 to 15% by weight, calculated to total of the powder composition.

In another form of the present invention, powder composition may be mixed into the oxidative dyeing composition prior to application onto hair. In this form of application of the present invention, the alkalizing agent may be comprised in the oxidative dyeing composition and therefore, in such a case, the powder composition may be free of alkalizing agent.

Thus, in another preferred form of carrying out the present invention is a process wherein a powder composition comprising at least one guanidine salt and at least one second alkaline salt selected from salts of carbonates, bicarbonates, phosphate and hydrogenphosphates with a cation selected from sodium, potassium or ammonium is mixed with two aqueous compositions wherein the first composition (Dyeing Composition) is an aqueous composition comprising at least one alkalizing agent and one or more hair dyes and the second composition (Oxidizing Composition) is an aqueous composition comprising at least one oxidizing agent and has an acidic pH, prior to application onto hair and is left on the hair for 1 to 45 min and is rinsed off from hair and hair is optionally dried.

The above disclosure on the powder composition is also valid for the powder composition used in the above process except for the alkalizing agent.

The aqueous Dyeing Composition of the above process comprises at least one alkalizing agent. Suitable ones are ammonia and compounds according to the general formula

R₁R₂R₃N

wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and preferably a mono or polyhydroxyalkyl. Further preferably R₁, R₂ and R₃ are same or different H, C₁ - C₄ alkyl, C₁ - C₄ monohydroxyalkyl, branched C₃ - C₄ monohydroxyalkyl or C₂ - C₄ polyhydroxyalkyl with the condition that at least one of R₁, R₂ and R₃ is not H and preferably a mono or polyhydroxyalkyl.

Suitable alkalizing agents are ammonia, monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures.

Preferred are ammonia, monoethanolamine, diethanolamine, triethanolamine and amino methyl propanol and their mixtures. The most preferred are ammonia, monoethanolamine and amino methyl propanol and their mixtures.

The concentration of total alkalizing agent in the compositions varies between 1 and 35%, preferably 1 and 30, more preferably 2.5 and 25 and most preferably 2.5 to 20% by weight calculated to the total the dyeing composition.

The aqueous composition comprises at least one hair dye. Suitable ones are oxidative dye precursor, coupling substance and direct dyes.

Suitable oxidative dyestuffs precursors are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono- and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylene-diamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropyl aniline, 2-chloro-p-phenylenediamine, 1-β-hydroxyethyl-2,5-diamino-4-chlorobenzene, 1-β-hydroxyethyl-2,5-diamino-4-methyl benzene, 2-methoxy-p-phenylenediamine, N,N-diethyl-p-phenylenediamine, 1-amino-4-β-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1,4-diamino isopropyl benzene and/or 1-amino-4-β-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diamino pyrazol-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4,5-diaminopyrazole, 1-(4-methylphenyl)methyl-4,5-diaminopyrazole, 1-methyl-3-phenyl-4,5-diaminopyrazole and the water-soluble salts. The use of the above mentioned oxidative dye precursors as mixture is also customary in hair coloring area.

The dyeing composition comprises preferably at least one coupling substance, which can be selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diamino- benzene, 1-amino-3-(2'-hydroxyethylamino)benzene, 1-amino-3-[bis(2'-hydroxy-ethyl) amino]benzene, α-naphthol, 4,6-dichlororesorcinol, 1,3-diamino-toluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2'-hydroxyethyl amino)benzene or the water-soluble salts thereof.

The total concentration of the oxidation dyestuff precursors and/or their water soluble salts and the coupling substances customarily ranges between 0.01 % and 10%, preferably 0.05% and 8 %, in particular 0.1 % to 6% by weight, calculated to the total hair dyeing composition.

The weight proportion of the named oxidative dye precursors to the coupling substances ranges between about 1 : 8 to 8 : 1, preferably about 1 : 5 to 5 : 1, in particular 1 : 2 to 2 : 1.

The aqueous dyeing composition comprises direct dyes of neutral, cationic and anionic character. Some examples to suitable cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12 Basic Red 22, Basic Red 51, Basic Red 76, Basic Violet 1, Basic Violet 2, Basic Violet 3, Basic Violet 10, Basic Violet 14 and Basic Yellow 57. According to the invention, suitable cationic dyestuffs are in principal those any available on the market for cosmetic hair colouring applications. For this purpose, special reference is made to the PCT application WO 95/15144 of Ciba-Geigy AG. The content of the PCT application WO 95/15144 is by reference incorporated here.

Examples to suitable direct acting anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No. 8, D&C Brown No. 1, D&C Green No. 5, D&C Green No. 8, D&C Orange No. 4, D&C Orange No. 10, D&C Orange No. 11, D&C Red No. 21, D&C Red No. 27, D&C Red No. 33, D&C Violet 2, D&C Yellow No. 7, D&C Yellow No. 8, D&C Yellow No. 10, FD&C Red 2, FD&C Red 40, FD&C Red No. 4, FD&C Yellow No. 6, FD&C Blue 1, Food Black 1, Food Black 2, Disperse Black 9 and Disperse Violet 1 and their alkali metal salts such as sodium, potassium.

Some examples to those suitable neutral dyes (HC dyes), so called nitro dyes, are HC Blue No.2, HC Blue No.4, HC Blue No.5, HC Blue No.6, HC Blue No.7, HC Blue No.8, HC Blue No.9, HC Blue No.10, HC Blue No.11, HC Blue No.12, HC Blue No.13, HC Brown No.1, HC Brown No.2, HC Green No.1, HC Orange No.1, HC Orange No.2, HC Orange No.3, HC Orange No.5, HC Red BN, HC Red No.1, HC Red No.3, HC Red No.7, HC Red No.8, HC Red No.9, HC Red No.10, HC Red No.11, HC Red No.13, HC Red No.54, HC Red No.14, HC Violet BS, HC Violet No.1, HC Violet No.2, HC Yellow No.2, HC Yellow No.4, HC Yellow No.5, HC Yellow No.6, HC Yellow No.7, HC Yellow No.8, HC Yellow No.9, HC Yellow No.10, HC Yellow No.11, HC Yellow No.12, HC Yellow No.13, HC Yellow No.14, HC Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

According to the invention, the dyeing composition comprises direct hair dyes at a total concentration of 0.01 to 10%, preferably 0.05 to 7.5%, more preferably 0.1 to 5% by weight calculated to the total the dyeing composition.

The aqueous dyeing compositions may be in the form of solutions, dispersions, gels and emulsions. Emulsions are the most preferred from.

The aqueous emulsion composition preferably comprises one or more fatty alcohol of the general formula

R₄-OH

wherein R₄ is a linear or branched, saturated or unsaturated alkyl chain with 12 to 22 C atoms and at least one emulsifier selected from anionic, non-ionic, cationic and amphoteric surfactants.

Suitable fatty alcohols are myristyl alcohol, cetyl alcohol, stearyl alcohol, oleyl alcohol, octyldodecanol. and behenyl alcohol and their mixtures. Preferred is the mixture of cetyl and stearyl alcohol also known as cetearyl alcohol.

The concentration of one or more fatty alcohols is in the range of 1 to 25%, preferably 2.5 to 20%, more preferably 5 to 15% and most preferably 5 to 12% by weight calculated to total of the dyeing composition.

The aqueous emulsion dyeing composition comprises at least one emulsifier selected from anionic, non-ionic, cationic and amphoteric surfactants. Preferred emulsifying surfactants are anionic, non-ionic and cationic ones and especially preferred are the mixture of anionic and non-ionic surfactants and mixture of cationic and non-ionic surfactants at any ratio. Preferred mixing ratio for the anionic - non-ionic emulsifying surfactant mixture and cationic - non-ionic emulsifying surfactant mixture is in the range of 5:1 to 1:5, more preferably 3:1 to 1:3 and especially 1:1, by weight. It should be noted that incompatibilities can arise when anionic and cationic surfactants are used as the mixture emulsifier which should be taken into account when selecting such a combinations.

In principal any anionic surfactant is suitable within the meaning of the present invention. Nonlimiting examples are anionic surfactants of the sulfate, sulfonate, carboxylate and alkyl phosphate type, especially, of course, those customarily used as emulsifiers, for example, the known C₁₀-C₁₈-alkyl sulfates, and in particular the respective ether sulfates, for example, C₁₂-C₁₄-alkyl ether sulfate, lauryl ether sulfate, especially with 1 to 4 ethylene oxide groups in the molecule, monoglyceride (ether) sulfates, fatty acid amide sulfates obtained by ethoxylation and subsequent sulfatation of fatty acid alkanolamides, and the alkali salts thereof, as well as the salts of long-chain mono- and dialkyl phosphates and their salts.

Further suitable surfactants of the carboxylate type are alkyl polyether carboxylic acids and the salts thereof of the formula

R₅-(OC₂H₄)ₙ-O-CH₂COOX,

wherein R₅ is a C₈-C₂₀-alkyl group, preferably a C₁₂-C₁₄-alkyl group, n is a number from 1 to 20, preferably 2 to 17, and X is H or preferably a cation of the group sodium, potassium, magnesium and ammonium, which can optionally be hydroxyalkyl-substituted, as well as alkyl amido polyether carboxylic acids of the general formula 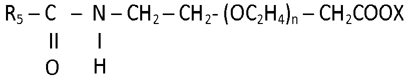 wherein R₅ and X have the above meanings, and n is in particular a number from 1 to 10, preferably 2.5 to 5.

Among the anionic surfactants most preferred are alkyl sulfates and/or alkyl ether sulfates and among them sodium lauryl or laureth sulfates and their mixtures are most preferred.

Suitable non-ionic surfactants are alkyl polyglucosides of the general formula

R₆-O-(R₇O)ₙO-Zₓ

wherein R₆ is an alkyl group with 8 to 18 carbon atoms, R₇ is an ethylene or propylene group, Z is a saccharide group with 5 to 6 carbon atoms, n is a number from 0 to 10 and x is a number between 1 and 5. Examples are decyl polyglucoside and cocoyl polyglucoside, both beeing commercially available.

Further nonionic surfactant components are, for example, long-chain fatty acid mono- and dialkanolamides, such as coco fatty acid monoethanolamide and myristic fatty acid monoethanolamide.

Further additionally useful nonionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "Pluronics^{R}".

Further nonionic surfactants as emulsifiers useful in the compositions according to invention are C₁₀-C₂₂-fatty alcohol ethoxylates. Especially suited are C₁₀-C₂₂-fatty alcohol ethers, the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Among the non-ionic surfactants mentioned above fatty alcohol ethoxylates and fatty acid alkanolamides and their mixtures at any weight ratio are the most preferred ones.

As a rule any mono alkyl quaternary ammonium surfactants is suitable for the compositions of the present invention as cationic emulsifying surfactant. With the term mono alkyl it is meant that quaternary ammonium surfactant includes only one alkyl chain which has more than 8 or more C atoms.

Preferably at least one mono alkyl quaternary ammonium surfactant is selected from the compounds with the general formula 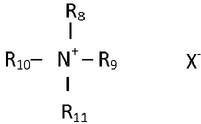 where R₈ is saturated or unsaturated, branched or straight alkyl chain with 8-22 C atoms or

R₁₂CONH(CH₂)ₙ

where R₁₂ is saturated or unsaturated, branched or straight alkyl chain with 7-21 C atoms and n has typical value of 1 - 4 or

R₁₂COO(CH₂)ₙ

where R₁₂ is saturated or unsaturated, branched or straight alkyl chain with 7-21 C atoms and n has typical value of 1 - 4, and

R₉, R₁₀ and R₁₁ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

Suitable cationic surfactants and or conditioning agents are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyl trimethyl ammonium chloride and stearamidopropyltrimethylammonium chloride.

Surfactants are comprised in the aqueous dyeing composition at a total concentration of 0.5 to 20%, preferably 1 to 15% and most preferably 2 - 12.5%, and most preferably 2 to 10% by weight, calculated to the total the dyeing composition.

Dyeing compositions may further comprise one or more polyol. Suitable ones are glycerine, phytantriol, panthenol, ethyleneglycol, polyethyleneglycols, propylene glycols such as 1,2 propylene glycol, 1,3-propylene glycol and polypropylene glycols. Concentration of one or more polyol is in the range of 0.1 to 15%, preferably 0.25 to 12.5%, more preferably 0.5 to 10% and most preferably 1 to 7.5% by weight calculated to the total of the composition.

The aqueous dyeing composition has a pH between 2 and 11, preferably 6 to 11, more preferably 6.8 to 11, most preferably 8 to 11 and in particular 9 to 10.5.

The oxidizing agents suitable for the oxidizing composition are hydrogen peroxide, urea peroxide, melamin peroxide or perborate salts. The preferred oxidizing agent is hydrogen peroxide, and preferably comprised at a concentration in a range of 1 to 12 % by weight calculated to the total composition.

The pH of the composition comprising at least one oxidizing agent is in the range of 1 to 5, preferably 1.5 to 4 and more preferably 1.5 to 3.

The compositions may additionally comprise an organopolysiloxane wherein at least one silicon atom is linked to an alkylene group having a hetero-atom, in particular a nitrogen atom, with a poly-(N-acyl alkyleneimine) units of the formula 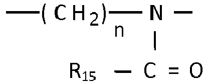 wherein n is a number from 1 to 5 and R₁₅ is hydrogen, a C₁-C₁₂-alkyl or cycloalkyl, aralkyl or aryl group.

Preferred organopolysiloxane polymers are those of the type disclosed in EP-A 640 643, in particular optionally quaternized aminoalkyl, in particular aminopropyl dimethyl polysiloxane/polyethyl oxazoline copolymers of the formula 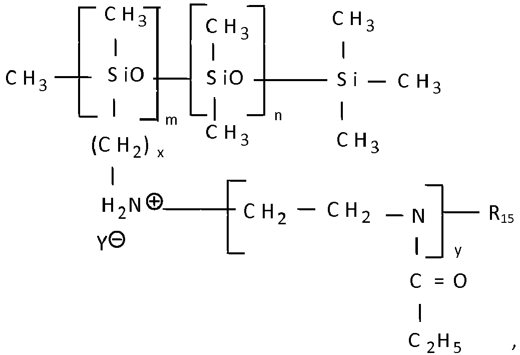 wherein m and n each are numbers from 20 to 10,000, in particular 50 to 7,000, especially 100 to 5,000, x is a number between 1 and 5, preferably 3, and y is a number from 5 to 30, R₁₅ is a C₁-C₁₂-alkyl or aryl group, in particular a methyl, ethyl or benzyl group, and Y- is an anion.

Especially suited are the organopolysiloxanes disclosed under the terms A-1, A-2 and A-3 on pages 12 to 13 of EP-A 640 643. The proportion of graft copolymers in the hair colouring compositions according to the invention ranges from 0.05 % to 5 %, preferably 0.1 % to 2.5 %, in particular 0.5 % to 1.5 % by weight, calculated to the total composition.

Another compound that may be comprised in the dyeing composition is a ceramide type of compounds according to the general formula 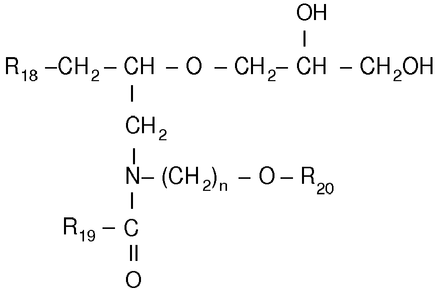 where R₁₈ and R₁₉ are independent from each other alkyl- or alkenyl group with 10 to 22 carbon atoms and R₂₀ is methyl, ethyl, n-propyl or isopropyl group. The concentration of the ceramide type of compound in colouring compositions of the present invention can be in the range of 0.01 to 2 and especially 0.01 to 1% by weight calculated to the total composition.

The dyeing compositions according to the present invention can further comprise one or more ubiquinone of the formula. 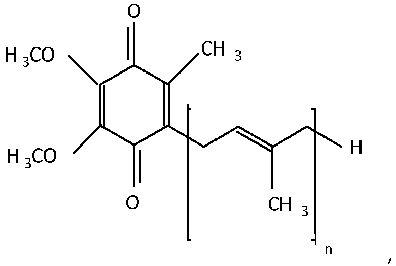 wherein n is a number from 1 to 10. The concentration of ubichinones in the compositions of the present invention can vary between 0.001% and 10% by weight, calculated to the total composition excluding the oxidizing agent.

The dyeing composition may certainly comprise compounds for accelerating (catalysts) the oxidative dyeing keratin fibres such as iodine salts i.e. potassium or sodium iodide and/or dihydroxy acetone.

The aqueous compositions of the present invention may further comprise any compounds found regularly in hair cosmetic compositions such as chelating agents, preservatives, reducing agents, fragrance, acids to adjust the pH and other suitable ones.

Following examples are to illustrate the invention but not to limit it.

### Example 1

| | % by weight |
|---|---|
| Ammonium bicarbonate | 17 |
| Guanidine carbonate | 40 |
| Sodium metasilicate | 8.5 |
| Silica | 34.5 |

The above composition was prepared by mixing all the components in a suitable vessel with each other.

25 g of above composition was mixed with 50 g of an aqueous oxidizing composition comprising hydrogen peroxide at a concentration of 9% by weight and applied onto hair and after leaving 30 min on the hair rinsed off. It was observed that hair was homogeneously bleached.

| **Component A (powder):** | |
|---|---|
| | % by weight |
| Ammonium bicarbonate | 15 |
| Guanidine carbonate | 32.5 |
| Silica | 52.5 |
| | |

| **Component B (emulsion):** | |
|---|---|
| | % by weight |
| Cetearyl alcohol | 20.0 |
| Ceteareth-20 | 10.0 |
| Isopropyl palmitate | 1.0 |
| Dimethicone | 1.0 |
| Polyquaternium-10 | 1.0 |
| Cetrimonium chloride | 1.0 |
| p-Toluenediamine sulphate | 1.25 |
| 1-Naphtthol | 1.25 |
| p-Aminophenol | 1.5 |
| 4-Amino-2-Hydroxytoluene | 1.25 |
| HC Yellow No.2 | 0.25 |
| 1,2 propylene glycol | 3.0 |
| Ammonia (25%) | 10.0 |
| Water | q.s. to 100 |

| **Component C (developer):** | |
|---|---|
| | % by weight |
| Hydrogen peroxide | 7.5 |
| Water | q.s. to 100 |

The mixing ratio of the components was 1:2:2, by weight (Parts A:Parts B:Parts C). The composition thus obtained was applied onto hair and after leaving 30 min on the hair, it was rinsed off. It was observed that hair was homogeneously coloured into a reddish colour tone.

## Claims

1. A powder composition for lightening and/or bleaching hair **characterised in that** it comprises at least one alkalizing agent, at least one guanidine salt and at least one second alkaline salt selected from salts of carbonates, bicarbonates, phosphate and hydrogenphosphates with a cation selected from sodium, potassium or ammonium.

2. The powder composition according to claim 1 **characterised in that** it does not comprise the persalts at a total concentration higher than 10% by weight, preferably 5% by weight and most preferably free of per salts, all values are calculated to the total of the powder composition.

3. The powder composition according to claims 1 and 2 **characterised in that** at least one guanidine salt is selected form guanidine carbonate, guanidine hydrochloride and guanidine phosphate preferably it is guanidine carbonate and preferably it is comprised at a concentration in the range of 5 to 90%, preferably 10 to 75%, more preferably 15 to 60% and most preferably 15 to 50% by weight calculated to the total of the powder composition.

4. The powder composition according to any of the preceding claims characterises in that at least one second alkaline salt selected from sodium carbonate, sodium bicarbonate, trisodium phosphate, disodium hydrogen phosphate, sodium dihydrogen phosphate, potassium carbonate, potassium bicarbonate, tripotassium phosphate, dipotassium hydrogen phosphate, potassium dihydrogen phosphate, ammonium carbonate, ammonium bicarbonate, triammonium phosphate, diammonium hydrogen phosphate and ammonium dihydrogen phosphate, preferably selected from sodium bicarbonate, sodium carbonate, potassium bicarbonate, potassium carbonate, ammonium carbonate and ammonium bicarbonate and more preferably selected from sodium bicarbonate, potassium bicarbonate and ammonium bicarbonate and in particular it is ammonium bicarbonate is the especially preferred alkaline salt which are preferably comprised at a concentration in the range of 5 to 90%, preferably 10 to 75%, more preferably 15 to 60% and most preferably 15 to 50% by weight calculated to the total of the powder composition.

5. The powder composition according to any of the preceding claims **characterised in that** at least one guanidine slat and at least one second alkaline salts is comprised in the powder composition at a weight in the range of 10:1 to 1:5, preferably 5:1 to 1:2, more preferably 3.1 to 1:2 and most preferably 2:1 to 1:1.

6. The powder composition according to any of the preceding claims **characterised in that** at least one alkalizing agent is sodium metasilicate and is comprised at a concentration in the range of 1 to 20%, preferably 2 to 15% more preferably 2.5 to 12.5 and most preferably 5 to 10% by weight calculated to the total of the powder composition.

7. The powder composition according to any of the preceding claims **characterised in that** it comprises a powder, inert carrier material preferwabyl selected from starch, diatomaceous earth, pyrogenic silicium dioxide.

8. A process for lightening and/or bleaching human hair wherein the powder composition according to claims 1 to 7 is mixed with an aqueous composition which has an acidic pH and comprises at least one oxidizing agent and is applied onto hair and after being left on the hair for 1 to 45 min is rinsed off from hair, and hair is optionally dried.

9. Kit for hair comprising two or more compositions wherein one of the compositions is the powder composition according to claims 1 to 7 and the other composition is an aqueous composition comprising at least one oxidizing agent and has an acidic pH.

10. Process for dyeing hair wherein a powder composition comprising at least one guanidine salt and at least one second alkaline salt selected from salts of carbonates, bicarbonates, phosphate and hydrogenphosphates with a cation selected from sodium, potassium or ammonium is mixed with two aqueous compositions wherein the first composition (Dyeing Composition) is an aqueous composition comprising at least one alkalizing agent and one or more hair dyes and the second composition (Oxidizing Composition) is an aqueous composition comprising at least one oxidizing agent and has an acidic pH, prior to application onto hair and is applied onto hair and is left on the hair for 1 to 45 min and is rinsed off from hair and hair is optionally dried.

11. The process according to claim 10 wherein the alkalizing agent in the dyeing composition is selected from ammonia and compounds according to the general formula
R₁R₂R₃N
wherein R₁, R₂ and R₃ are same or different H, C₁ - C₆ alkyl, C₁ - C₆ monohydroxyalkyl, branched C₃ - C₆ monohydroxyalkyl or C₂ - C₆ polyhydroxyalkyl with the condition that at least one of R₁, R₂ and R₃ is not H and preferably a mono or polyhydroxyalkyl, preferably R₁, R₂ and R₃ are same or different H, C₁ - C₄ alkyl, C₁ - C₄ monohydroxyalkyl, branched C₃ - C₄ monohydroxyalkyl or C₂ - C₄ polyhydroxyalkyl with the condition that at least one of R₁, R₂ and R₃ is not H and preferably a mono or polyhydroxyalkyl.

12. The process according to claims 10 and 11 wherein at leats one hair dye is selected from oxidative dye precursors and direct dyes.

13. The process according to claims 10 to 12 wherein the alkalizing agent in the hair dyeing composition is selected from ammonia, monoethanolamine, diethanolamine, triethanolamine, monoethanol methylamine, monoethanoldimethylamine, di-ethanolmethylamine, monoethanolethylamine, monoethanoldiethylamine, diethanolethylamine, monoethanolpropylamine, monoethanoldipropylamine, diethanolpropylamine, monoethanolbutylamine, diethanolbutylamine and amino methyl propanol and their mixtures and comprised at a total concentration between 1 and 35%, preferably 1 and 30, more preferably 2.5 and 25 and most preferably 2.5 to 20% by weight calculated to the total the dyeing composition.

14. The process according to claims 10 to 13 wherein the dyeing composition is an emulsion and comprises one or more fatty alcohol of the general formula
R₄-OH
wherein R₄ is a linear or branched, saturated or unsaturated alkyl chain with 12 to 22 C atoms and at least one emulsifier selected from anionic, non-ionic, cationic and amphoteric surfactants.

15. The process according to claims 10 to 14 wherein the oxidizing composition comprises at least one oxidizing agent selected from hydrogen peroxide, urea peroxide, melamin peroxide or perborate salts at a concentration in a range of 1 to 12 % by weight calculated to the total oxidizing composition and has a pH in the range of 1 to 5, preferably 1.5 to 4 and more preferably 1.5 to 3.
